# EUROPÄISCHE PATENTSCHRIFT

(11) **EP 3 810 561 B1**
(45) Veröffentlichungstag und Bekanntmachung des Hinweises auf die Patenterteilung: **24.01.2024**
(21) Anmeldenummer: 19742131.6
(22) Anmeldetag: 14.06.2019
(51) Int. Cl.: C05G 5/30, C05C 9/00, B01J 2/00

(54) **VERWENDUNG VON ÜBERKRITISCHEM CO2 ALS LÖSUNGSMITTEL FÜR ORGANISCHE POLYMERE IN EINEM BESCHICHTUNGSVERFAHREN UND EINER BESCHICHTUNGSANLAGE FÜR HARNSTOFFHALTIGES GRANULAT**
USE OF SUPERCRITICAL CO2 AS SOLVENT FOR ORGANIC POLYMERS IN A METHOD AND APPARATUS FOR COATING UREA-CONTAINING GRANULES
UTILISATION DE CO2 SUPERCRITIQUE EN TANT QUE SOLVANT POUR DES POLYMÈRES ORGANIQUES DANS UN PROCÉDÉ ET UNE INSTALLATION DE REVÊTEMENT POUR UN GRANULÉ CONTENANT DE L'URÉE

(30) Priorität: 20.06.2018 DE 102018210030
(43) Veröffentlichungstag der Anmeldung: 28.04.2021
(73) Patentinhaber: thyssenkrupp Uhde GmbH, 44141 Dortmund (DE); thyssenkrupp AG, 45143 Essen (DE)
(72) Erfinder: MAKHYNYA, Yevgeny, 45481 Mülheim an der Ruhr (DE); EL HAWARY, Tarek, 59439 Holzwickede (DE)
(74) Vertreter: thyssenkrupp Intellectual Property GmbH
(86) Internationale Anmeldenummer: PCT/EP2019/065758
(87) Internationale Veröffentlichungsnummer: WO 2019/243204

(56) Entgegenhaltungen:
- WO-A1-2019/063681
- US-A- 5 766 637

## Beschreibung

Die Erfindung betrifft ein Verfahren zur Beschichtung von harnstoffhaltigen Partikeln mit biologisch abbaubaren Polymeren, einen Anlagenkomplex zur Beschichtung von harnstoffhaltigen Partikeln mit bioabbaubaren Polymeren und die Verwendung des erfindungsgemäßen Anlagenkomplex zur Beschichtung von harnstoffhaltigen Partikeln mit bioabbaubaren Polymeren.

Im Hinblick auf das weltweite Bevölkerungswachstum kommt der Entwicklung von flexiblen und effizienten Düngern eine große und wachsende Bedeutung zu. Dabei spielt nicht nur der Dünger selbst, d.h. die chemische Zusammensetzung, sondern auch die Verarbeitungen in transportfähige Gebinde und die Ausbringung auf dem das Feld eine Rolle. Die größte Bedeutung kommt hierbei sicherlich der Granulierung zu gleichmäßigen, in Größe und Beschaffenheit gleichen Partikeln, zu. Wichtige Parameter sind hierbei geringe Staubbildung, Festigkeit, niedrige Aggregationstendenz, homogene Größe, Lagerfähigkeit und Beständigkeit. Eine etablierte Granulationstechnik ist beispielsweise die Fließbettgranulation, welche gegenüber beispielsweise den Prill- und Pastilliertechniken, verbesserte Partikeleigenschaften aufweist.

Einen sehr großen Anteil an der weltweiten Düngerproduktion entfällt auf harnstoffhaltige Dünger. Diese wasserlöslichen Dünger transformieren sich im Boden zu Ammoniumsalzen bzw. Nitraten und stellen einen wichtigen Basisdünger dar. Diese harnstoffhaltigen Dünger können mit weiteren Elementen wie Kalium, Mangan, Phosphaten, Schwefel, Schwefelverbindungen, Selen, Kalzium, u.a. kombiniert werden.

Neben einer guten Verfügbarkeit des Düngers im Boden ist auch eine gute Bestimmbarkeit und Reproduzierbarkeit der Nährstoffabgabe an die Pflanze von großer Bedeutung. Hierbei kann es insbesondere am Anfang der Düngung und bei Dosierungsfehlern zu einem Überschuss des Düngers kommen. Dieser Überschuss kann die Pflanzen schädigen und sogar zu einem Absterben führen. Diese Verluste des Düngers sind auch für die Probleme mit Böden und Grundwasser zuständig. Zur Lösung dieser Problematik sind beschichtete Düngergranulate bekannt. Dabei wird in der Regel ein Kern aus düngerhaltigem Granulat mit einer oder mehreren Ummantelungen versehen. Je nach Anwendungsprofil kann eine sehr schnelle Auflösung, beispielsweise durch ein wasserlösliches Polymer wie Stärke oder Polyethylenoxid erfolgen. Ist eine langsamere Auflösung und Abgabe des Düngers wünschenswert, so eignen sich Ummantelungen aus organischen Polymeren, insbesondere biologisch abbaubare Polymerummantelungen. Die Polymerschicht verhält sich ähnlich einer Membran und ermöglicht ein Eindringen von Wasser/Wasserdampf in die Partikel. Die im Wasser gelösten Dünger verlassen anschließend durch Diffusion und Osmose die zurückbleibende "Polymerhülle", und ermöglichen somit eine gezielte Abgabe des Düngers an den Boden und die Pflanze. Die biologische Abbaubarkeit einer polymerhaltigen Beschichtung im Boden (beispielsweise im Zeitraum von ca. 1-2 Jahre) kann beispielsweise mittels der DIN EN ISO 14855-1:2013-04 oder ASTM D 5338 bestimmt werden. Je nach Anwendungsbereich können auch mehrere Beschichtungen nacheinander aufgetragen werden. Beispiele für beschichte Düngegranulate finden sich beispielsweise in der DE 10 2005 028 016 A1. Eine ausführliche Beschreibung zur Bestimmung der biologischen Abbaubarkeit von organischen Polymeren findet sich beispielsweise unter "Castia Bastioli, Handbook of Biodegradable Polymers, Rapra Technology Limited, 2005, ISBN: 1-85957-389-4, Kapitel 5, Seiten 145-181" und in den beschriebenen Normen ISO 14852, ISO 14851, ISO 14855, ISO 14855 Amendment 1, EN 13432, DIN V54900, ASTM D6002-96, ASTM D6400-99.

Die Auswahl eines geeigneten Polymers für das entsprechende Düngemittelgranulat ist jedoch mit einigen Schwierigkeiten verbunden. So kann das Polymer beispielsweise wie in der US 5,766,637 A in Spalte 1 beschrieben, aufgeschmolzen, polymerisiert oder gelöst werden. Die Aufbringung eines Polymers in einem Lösungsmittel ist hierbei sicherlich die gebräuchlichste und technisch einfachste Anwendung.

Da die Düngergranulate anschließend auf dem Boden ausgebracht werden und sich mit anderen Teilen der Beschichtung und/oder verwendeten Lösungsmitteln auch zumindest theoretisch in den zu düngenden Pflanzen anreichern können, dürfen nur ausgewählte, nicht gesundheits- und/oder umweltschädliche Lösungsmittel verwendet werden. Geeignete Lösungsmitte für Polymere, beispielsweise chlorhaltige Lösungsmittel wie DCM (Dichlormethan) oder TCM (Trichlormethan) dürfen in der Landwirtschaft überhaupt nicht verwendet werden. Naturgemäß lässt sich im Bereich der organischen Lösungsmittel diese Einstufung in gesundheitsschädlich oder nicht gesundheitsschädlich nur schwer vornehmen. Zudem ist im Zuge der immer strengeren Umweltregularien von einem Anstieg der Einstufung von Lösungsmitteln als "gesundheitsschädlich" auszugehen. Neben der Anreicherung im Boden setzten organische Lösungsmittel, insbesondere flüchtige organische Lösungsmittel "VOCs" (volatile organic compounds), auch besondere Anforderungen an den eigentlichen Beschichtungsvorgang. Hierbei müssen die Bildung explosiver Gemische genau wie die Exposition der Mitarbeiter mit berücksichtigt werden.

US 4,019,890 A offenbart ein Verfahren zur Beschichtung eines Düngergranulates mit einem thermoplastischen Polymer. Als Polymere kommen Polyolefine wie beispielsweise Polyethylen oder Polypropylen, als Lösungsmittel Tetrachlorethylen, Toluol, Xylen oder Trichlorethylen in Frage.

WO 03/082003 A2 offenbart einen partikulären, harnstoffstoffhaltigen Dünger, welcher eine verzögerte Abgabe von Stickstoff im Boden ermöglicht. Der Dünger umfasst ein Kernmaterial und eine Beschichtung auf Basis eines Harnstoff-Formaldehyd Polymers. Das Polymer wird über einen Binder, beispielsweise ein Harnstoff-Formaldehyd Harz, an dem Kernmaterial gebunden.

US 5,766,637 A offenbart die Mikrokapsulierung eines Kernmaterials in einer Polymermatrix. Das Polymer wird mit dem Kernmaterial vermischt und die Mischung in überkritischen CO₂ gelöst. Das resultierende Produkt enthält das fein verteilte Kernmaterial in der geschwollenen Polymermatrix.

EP 0 706 821 A1 offenbart ein Verfahren zum Beschichten von Mikropartikeln. Das Verfahren beinhaltet den Einschluss einer aktiven Verbindung in einer Ummantelung. Das Verfahren beinhaltet das Suspendieren der aktiven Substanz in einem überkritischen Fluid enthaltend das ummantelnde Material.

DE 10 2005 028 016 A1 offenbart ein Düngemittel, welches eine Beschichtung mit einem biologisch oder hydrolytisch abbaubaren Oligomer oder Polymer aufweist, welches eine kontrollierbare Wirkstofffreigabe ermöglicht, welche im Vergleich zu nicht beschichteten Düngemitteln zeitlich stark verzögert abläuft.

Die vorliegende Erfindung hat die Aufgabe beschichtete Düngergranulate bereitzustellen, die ohne den Einsatz von umwelt- und/oder gesundheitsschädlichen Lösungsmitteln hergestellt werden können. Gleichzeitig sollen möglichst wenig kostenintensive zusätzliche apparative Umbaumaßnahmen erforderlich sein und ein zusätzlicher logistischer Aufwand vermieden werden.

Die Aufgabe der Erfindung wird überraschenderweise durch ein Verfahren zur Beschichtung von harnstoffhaltigen Granulat mit organischen Polymeren gemäß Anspruch 1 gelöst. Weitere vorteilhafte Ausgestaltungen finden sich in den abhängigen Ansprüchen.

Die Erfindung umfasst des Weiteren einen Anlagenkomplex zur Beschichtung von harnstoffhaltigen Partikeln mit bioabbaubaren Polymeren gemäß Anspruch 13. Weitere vorteilhafte Ausgestaltungen finden sich in den jeweiligen abhängigen Ansprüchen.

Die Erfindung umfasst des Weiteren die Verwendung des erfindungsgemäßen Anlagenkomplexes zur Beschichtung von harnstoffhaltigen Partikeln mit bioabbaubaren Polymeren gemäß Anspruch 18.

Das erfindungsgemäße Verfahren zur Beschichtung von harnstoffhaltigen Granulat mit organischen Polymeren umfasst mindestens die folgenden Schritte. In einem ersten Schritt wird überkritisches Kohlendioxid bereitgestellt. Ein Vorteil des Verfahrens liegt darin, dass ein vorhandener CO₂ Kompressor genutzt werden kann und als Teilstrom das CO₂ bei den vorhandenen Drücken ableitet. Bevorzugt wird das CO₂ in einem Ammoniak-Harnstoff Komplex bereitgestellt. Der Ausdruck Ammoniak-Harnstoff-Komplex bezeichnet im Sinne der Erfindung die Kombination einer Anlage zur Ammoniaksynthese mit einer Anlage zur Harnstoffsynthese. Die Bereitstellung des überkritischen Kohlendioxids erfolgt dabei über das Komprimieren vom gasförmigen Kohlendioxid und das anschließende Kondensieren des Kohlendioxids und das Erhalten vom flüssigen Kohlendioxid. Im nächsten Schritt wird der Druck und/oder die Temperatur über den kritischen Punkt von Kohlendioxid erhöht und ein überkritisches Kohlendioxid erhalten. Bevorzugt wird das überkritische Kohlendioxid folgendermaßen bereitgestellt. In einem ersten Schritt wird gasförmiges Kohlendioxid komprimiert. Bevorzugte Druckbereiche umfassen 20 bar bis 35 bar. Anschließend wird das voranstehend komprimierte Kohlendioxid bevorzugt in einem Kondensator, beispielsweise mit Hilfe eines geeigneten Kältemittels (z.B. in einer NH₃ Kälteanlage, für weitere Kältemittel siehe beispielsweise VDI Wärmeatlas, ISBN 978-3-642-19980-6) kondensiert. Bevorzugt wird das Kohlendioxid dabei auf Temperaturen zwischen - 15°C und -35 °C abgekühlt. Das flüssige Kohlendioxid wird im nächsten Schritt bevorzugt einer Komprimierung und einer Temperaturerhöhung unterzogen. Dabei werden sowohl der Druck als auch die Temperatur über die kritischen Werte (Für reines Kohlendioxid :T_{C} = 31,00 °C p_{C} = 76,262 bar aus Holleman, Wiberg, Lehrbuch der Anorganischen Chemie, 102 Auflage, 2007, Seite 894 (ISBN 978-3-11-017770-1), je nach Reinheit des Kohlendioxids kann die kritische Temperatur T_{C} und der kritische Druck p_{C} hiervon abweichen) erhöht und überkritisches Kohlendioxid erhalten. Die Zwischenstufe über den obigen Kondensationsschritt im Vergleich zur direkten Komprimierung des gasförmigen Kohlendioxids ist eine apparativ einfachere, energiesparende und zuverlässigere anschließende Komprimierung und Temperaturerhöhung. In einem zweiten Schritt wird ein organisches Polymer, bevorzugt ein bioabbaubares organisches Polymer, in dem überkritischen Kohlendioxid gelöst und eine polymerhaltige Lösung erhalten. Der Ausdruck "polymerhaltige Lösung" umfasst im Sinne der Erfindung auch polymerhaltige Suspensionen und Emulsionen. Der Ausdruck "bioabbaubares organisches Polymer" umfasst Polymere die gemäß mindestens einer oder mehrerer der folgenden Normen ISO 14852, ISO 14851, ISO 14855, ISO 14855 Amendment 1, EN 13432, DIN V54900, ASTM D6002-96, ASTM D6400-99 unter zu mindestens teilweiser Auflösung ihrer chemischen Struktur abgebaut werden können. Bevorzugt umfasst der Ausdruck "bioabbaubares organisches Polymer" organische Polymere die eine Abbaubarkeit nach EN 13432 von mehr als 50 %, besonders bevorzugt die Norm EN 13432 mit mindestens 90 % erfüllen. Eine ausführliche Beschreibung zur biologischen Abbaubarkeit findet sich wie voranstehend beschrieben unter "Castia Bastioli, Handbook of Biodegradable Polymers, Rapra Technology Limited, 2005, ISBN: 1-85957-389-4, Kapitel 5, Seiten 145-181". In einem folgenden Schritt erfolgt das Vermischen der polymerhaltigen Lösung mit einem harnstoffhaltigen Granulat und die Erniedrigung der Temperatur und/oder des Drucks unterhalb des kritischen Punkt von Kohlendioxid.

Bevorzugt erfolgt der Schritt des "Vermischen" der Granulate mit der polymerhaltigen Lösung über ein Besprühen, besonders bevorzugt in einem Trommelcoater oder in einem Wirbelschichtcoater. Dabei erfolgt unter abrupter Senkung des Druckes und der Temperatur eine Anhaftung und Erstarren des Polymers auf dem Granulat statt. Das harnstoffhaltige Granulat weißt mittlere Partikelgrößen von 0,5 mm bis 8 mm, bevorzugt 1 mm bis 6 mm, besonders bevorzugt von 2 mm bis 4 mm auf. Die Vermischung der polymerhaltigen Lösung mit einem harnstoffhaltigen Granulat sowie die weitere Temperaturabsenkung und/oder Druckabsenkung können im Rahmen der Erfindung sowohl nachfolgend als auch gleichzeitig erfolgen. Eine näherungsweise gleichzeitige Vermischung und beispielsweise Druckabsenkung lässt sich beispielsweise durch Aufsprühen der polymerhaltigen Lösung auf das harnstoffhaltige Granulat realisieren. Dementsprechend kann die Phasenumwandlung des Kohlendioxids vom überkritischen Aggregatzustand in den gasförmigen Aggregatzustand beispielsweise simultan mit dem Aufsprühen oder nachfolgend nach einer Vermischung beispielsweise durch Vermengen oder Rühren erfolgen. Mit der Phasenumwandlung des Kohlendioxids (überkritisch zu gasförmig) konnte eine parallele Anhaftung bzw. Beschichtung des Polymers auf der Oberfläche des harnstoffhaltigen Granulats festgestellt werden, dieser Prozess ist ähnlich dem Verdampfen des Lösungsmittels beim Besprühen unter Normaldruck. In diesem Vermischungsschritt, beispielsweise durch Aufsprühen oder Einrühren, wird im Zuge der Druckerniedrigung ein beschichtetes harnstoffhaltiges Granulat und gasförmiges Kohlendioxid erhalten. Das harnstoffhaltige beschichtete Granulat weißt mittlere Partikelgrößen von 0,5 mm bis 8 mm, bevorzugt 1 mm bis 6 mm, besonders bevorzugt von 2 mm bis 4 mm auf.

Das Verfahren ist bevorzugt dadurch gekennzeichnet, dass das überkritische Kohlendioxid in einem verbundenen industrielen Anlagenkomplex bereitgestellt wird. Die Verbindung kann über übliche Verbindungselemente, Rohre, Kompressoren, Pumpen usw. hergestellt werden. Der Ausdruck "industriele Anlagenkomplex" umfasst im Sinne der Erfindung Harnstoffsyntheseanlagen, Kokereianlagen, Raffinerieanlagen und/oder einen Ammoniak-Harnstoff-Komplex. Das in diesen Anlagen anfallende Kohlendioxid kann direkt (bevorzugt nach Reinigung) zur Herstellung von überkritischem Kohlendioxid verwendet werden, lange Transportwege und Transportvorrichtungen fallen weg. Der Ausdruck "überkritischen Kohlendioxid" umfasst im Sinne der Erfindung auch überkritische Lösungsmittel mit einem Kohlendioxidgehalt von ≥ (größer/gleich) 50 Gew. % (Gewichtsprozent) Kohlendioxid.

Bevorzugt umfasst das organische Polymer wie voranstehend beschrieben biologisch abbaubare Polymere.

In einer bevorzugten Ausführungsform umfasst das biologisch abbaubare Polymer Polylactide (PLA), Polyglykole, Polycaprolactone, Poly(hydroxybuttersäure), Poly(hydroxyvalerinsäure), Polyalkylterephthalate, Polyanhydride, Poly(1,4-dioxan-2,5-dione), Polyaminosäuren, Peptide, Polysaccharide, Cellulosester, Cellulosehydrat, Celluloseacetat, Carboxymethylcellulose, Lignin, Polyhydroxyfettsäuren, Stärke, biologisch abbaubare Polyester, biologisch abbaubare Polyamide, biologisch abbaubare Polyimide, Polyhydroxyalkanoate und Polybuthylensuccinate (PBS), Amylose, Amylopektin, und/oder Gemische, Oligomere, Derivate und/oder Copolymere davon.

Bevorzugt enthält die polymerhaltige Lösung größer 20 Gew. % bis 70 Gew. % biologisch abbaubare Polymere, besonders bevorzugt 40 Gew. % bis 60 Gew. % biologisch abbaubare Polymere.

Bevorzugt wird das gasförmige Kohlendioxid ins Verfahren zurückgeführt und in überkritisches Kohlendioxid umgewandelt. Diese Kreislaufführung senkt deutlich den Kohlendioxidverbrauch und die Energie- sowie Betriebskosten innerhalb des erfindungsgemäßen Verfahrens. Alternativ ist auch eine Entsorgung des CO₂ möglich. Dies richtet sich vornehmlich nach den lokalen Gegebenheiten wie Umweltvorschriften, Betriebskosten und technischer Aufwand der Rückführung.

Bevorzugt wird das gasförmige Kohlendioxid und/oder das überkritische Kohlendioxid in einem verbundenen industrielen Anlagenkomplex bereitgestellt, besonders bevorzugt in einer Harnstoffsynthese-Anlage, Ammoniaksynthese-Anlage, Kokereianlage, Raffinerieanlagen und/oder in einem Ammoniak-Harnstoff-Komplex, insbesondere bevorzugt einem Ammoniak-Harnstoff-Komplex. Der Ausdruck Ammoniak-Harnstoff-Komplex bezeichnet im Sinne der Erfindung die Kombination einer (bevorzugt örtlich verbundenen) Anlage zur Ammoniaksynthese mit einer Anlage zur Harnstoffsynthese. Die Ammoniaksynthese-Anlage stellt dabei den Ammoniak und zu mindestens Teile des Kohlendioxids zur Verfügung.

Bevorzugt erfolgt das voranstehend beschriebene Kondensieren mithilfe eines Kühlmittels, insbesondere Ammoniak, besonders bevorzugt mit Ammoniak aus einer angeschlossenen Ammoniak-Kälteanlage. Hierbei kann beispielsweise der in der angeschlossenen Ammoniakanlage anfallende Ammoniak als Kühlmittel verwendet werden.

Insbesondere bevorzugt wird das flüssige Kohlendioxid einen Flash Verfahren für die Entfernung und/oder Ausgasen Inerter und nicht kondensierbarer Gase unterzogen.

Bevorzugt erfolgt das Komprimieren des gasförmigen Kohlendioxids in einer ersten Stufe A in einer LP(Niederdruck)-Kompressionsstufe (bevorzugte Druckbereiche von 5 bar bis 10 bar) und in einer zweiten Stufe B in einer HP(Hochdruck)-Kompressionsstufe (bevorzugte Druckbereiche von 20 bar bis 30 bar). Besonders bevorzugt befinden sich die LP(Niederdruck)-Kompressionsstufe und die HP(Hochdruck)-Kompressionsstufe in einem Gehäuse.

Besonders bevorzugt sind eine Kälte-/Wärmerückgewinnung und/oder Kühler in den folgenden Verfahrensschritten vorgesehen:
- vor der ersten Stufe A;
- zwischen der ersten Stufe A und der zweiten Stufe B; und/oder
- nach der zweiten Stufe B.

Der Ausdruck "Kälte-ZWärmerückgewinnung" umfasst im Sinne der Erfindung dem Fachmann gebräuchliche Wärmetauscher und ähnliche Vorrichtungen.

In einer bevorzugten Ausführungsform enthält das harnstoffhaltige Granulat und/oder das beschichtete harnstoffhaltige Granulat Ammoniumsalze, Nitrate, Phosphate, Schwefel, Kalium, Kalzium, bevorzugt Harnstoff, Ammoniumsulfat, Ammoniumnitrat, Phosphate, Schwefel und/oder Gemische davon.

Bevorzugt erfolgt das Vermischen der polymerhaltigen Lösung mit dem harnstoffhaltigen Granulat in einem oder mehreren Beschichtern, bevorzugt Trommelcoater, Wirbelschichtcoater, und/oder Wirbelschichtcoater mit tangentialem Sprühen.

Die Erfindung umfasst des Weiteren ein Anlagenkomplex zur Beschichtung von harnstoffhaltigen Granulat mindestens umfassend, eine Harnstoffsynthese-Anlage, optional eine angeschlossene Ammoniaksynthese-Anlage und eine Anlage zur Beschichtung von harnstoffhaltigen Granulat/Partikeln. Bevorzugt sind die Ammoniaksynthese-Anlage und die Harnstoffsynthese-Anlage in einem Ammoniak-Harnstoff-Komplex zusammengefasst. Der Ausdruck Ammoniak-Harnstoff-Komplex bezeichnet dabei im Sinne der Erfindung mindestens zwei Anlagen - jeweils zur Ammoniak und Harnstoff Synthese-, die meist in einer unmittelbarer Nähe zu einander sich befinden und meist unter Verwendung von gemeinsamen Offsites und Utilities betrieben werden. Bevorzugt wird das Kohlendioxid in der Ammoniaksynthese-Anlage bereitgestellt und in der Harnstoffsynthese-Anlage komprimiert und in überkritisches CO₂ umgewandelt. Der Ausdruck "Granulat" umfasst im Sinne der Erfindung Partikel, Granulate, Agglomerate, bevorzugt im Bereich eines mittleren Partikeldurchmessers von 0,1 mm bis 10 mm. Ammoniaksynthese-Anlagen sind dem Fachmann bekannt, eine Erzeugung des Ammoniaks erfolgt bevorzugt im Grundsatz wie beispielsweise bei Holleman, Wiberg, Lehrbuch der Anorganischen Chemie, 102 Auflage, 2007, Seiten 662-665 (ISBN 978-3-11-017770-1) beschrieben, basierend auf dem "Haber-Bosch-Verfahren" aus den Elementen gemäß Gleichung (1):

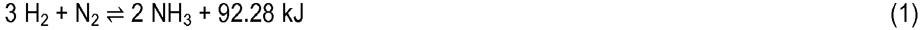

Das Edukt Stickstoff (N₂) kann beispielsweise durch Tieftemperaturluftzerlegung oder durch Reduktion von Sauerstoff in der Luft gewonnen werden. Der Wasserstoff wird bevorzugt über den "Steam-Reforming-Prozess" gemäß Gleichung (2) erhalten:

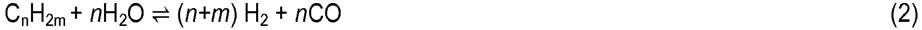

In der anschließenden "Kohlendioxid-Konvertierung" erfolgt eine weitere Umsetzung gemäß Gleichung (3):
Das gemäß Gleichung (3) entstehende Kohlendioxid (CO₂) dient bevorzugt Kohlendioxidquelle zur Gewinnung des überkritischen Kohlendioxids wie voranstehend und nachfolgend beschrieben. Reaktionsbedingungen, geeignete Katalysatoren sowie alternative Verfahren finden sich beispielsweise auf den Seiten 663 bis 664 des voranstehend zitierten Holleman, Wiberg, Lehrbuch der Anorganischen Chemie.

Der erfindungsgemäße Anlagenkomplex umfasst des Weiteren mindestens eine Zuleitung für überkritisches Kohlendioxid aus der Harnstoffsynthese-Anlage und eine Zuleitung für ein bioabbaubares Polymer. Des Weiteren ist eine Mischvorrichtung für überkritisches Kohlendioxid und des biologisch abbaubaren Polymers umfasst, welche mit der Zuleitung für das überkritische Kohlendioxid und der Zuleitung des biologisch abbaubaren Polymers verbunden ist. Der Ausgang der Mischvorrichtung sowie eine Zuleitung für ein harnstoffhaltiges Granulat sind mit einem Beschichter verbunden. Der Beschichter weist einen ersten Ausgang für gasförmiges Kohlendioxid und einen zweiten Ausgang für ein beschichtetes Produkt aus harnstoffhaltigen Granulat und einer biologisch abbaubaren Polymerummantelung auf. Der Beschichter umfasst bevorzugt im Sinne der Erfindung bevorzugt druck- und temperaturbeständige Rühr- und Mischvorrichtung, Coater und Sprühvorrichtungen. Der Ausdruck "verbunden" umfasst im Sinne der Erfindung dem Fachmann bekannte Rohre, Verbindungselemente, Pumpen, Kompressoren, usw. welche einen Transport von Gasen, Flüssigkeiten, Feststoffen sowie Gemischen davon ermöglichen. Bevorzugt ist eine mit einer Ammoniak-Syntheseeinheit als Teil eines Ammoniak-Harnstoff-Komplex und/oder eine Harnstoffgranulationsanlage enthalten. Die Harnstoffgranulationsanlage umfasst bevorzugt Fließbettgranulations- und/oder Prillanlagen. Die voranstehende Kombination ermöglicht eine lokale Herstellung des Beschichteten harnstoffhaltigen Granulats an einem Ort und ohne zusätzliche Logistikaufwendungen.

Der erste Ausgang für gasförmiges Kohlendioxid des Beschichters ist mit einem Staubwäscher, Staubabscheiderund/oder Zyklon verbunden. Mit Hilfe der voran genannten Vorrichtungen lassen sich die Staubemissionen bei einer Abgabe der des Kohlendioxids an die Atmosphäre (ohne Rezyklisierung des Kohlendioxids) reduzieren.

Bevorzugt ist eine Kohlendioxid-Komprimierungsstufe innerhalb der HarnstoffsyntheseAnlage enthalten. Bevorzugt erfolgt die Bereitstellung und Erzeugung des Ammoniaks in einer Ammoniaksynthese-Anlage. Diese ermöglicht eine Bereitstellung von überkritischem Kohlendioxid in der Harnstoffstoffsynthese-Anlage. Die Bereitstellung kann ohne größere Umbaumaßnahmen in der Harnstoffsynthese-Anlage erfolgen und die dort vorhandenen Druck- und Komprimierungsvorrichtungen. Bevorzugt kann die Bereitstellung des überkritischen Kohlendioxids sowohl mit Elementen der Harnstoffsynthese-Anlage als auch der Ammoniaksynthese-Anlage erfolgen.

Bevorzugt umfasst die die Kohlendioxid-Komprimierungsstufe innerhalb der Harnstoffsynthese-Anlage mindestens eine Kohlendioxid CO₂-Zuleitung verbunden mit einer (mit von der ersten zur vierten Stufe ansteigenden Druck) ersten Kompressionsstufe (bevorzugte Druckbereiche von 5 bar bis 10 bar) und eine zweite Kompressionsstufe (bevorzugte Druckbereiche von 20 bar bis 30 bar) verbunden mit der ersten Kompressionsstufe. Eine dritte Kompressionsstufe (bevorzugte Druckbereiche von 70 bar bis 90 bar) verbunden mit der zweiten Kompressionsstufe und eine vierte Kompressionsstufe (bevorzugte Druckbereiche von 140 bar bis 180 bar) verbunden mit der dritten Kompressionsstufe. Die Kompressionsstufen beinhalten dabei sowohl einzelne Kompressoren als auch Kombinationen von jeweiligen Niederdruck- (LP) und Hochdruckstufen (LP) innerhalb eines Gehäuses. Bevorzugt bilden die erste und zweite Kompressionsstufe eine Niederdruck- (LP) und Hochdruckstufen (LP) innerhalb eines ersten Gehäuses. Weiterhin bevorzugt bilden die dritte und vierte Kompressionsstufe eine Niederdruck- (LP) und Hochdruckstufe (LP) innerhalb eines zweiten Gehäuses. Bevorzugt werden die Gehäuse (erstes und/oder zweites Gehäuse) von einer Turbine angetrieben. In einer optionalen Ausgestaltung ist eine erste Verbindungsleitung zu einer optionalen Harnstoffsynthese-Anlage verbunden mit der vierten Kompressionsstufe enthalten. Diese ermöglicht eine Verwendung des komprimierten Kohlendioxids in der Synthese von Harnstoff gemäß der vereinfachten Gleichungen (5) und (6): [aus Ullmann's Encyclopedia of Industrial Chemistry, Introduction, 2010, DOI: 10.1002/14356007.a27_333.pub2]

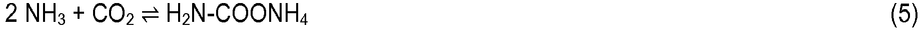

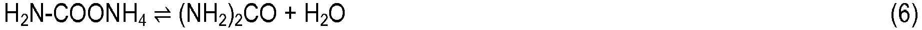

Der erfindungsgemäße Anlagenkomplex umfasst eine Abzweigung zwischen der zweiten Kompressionsstufe und der dritten Kompressionsstufe. Ausgehend von der Abzweigung führt eine zweite Verbindungsleitung zu einer Entgasungsanlage, wobei die zweite Verbindungsleitung eine vor der Entgasungsanlage eine Kälteanlage durchläuft (durchgehend verbunden ist). Die Kälteanlage ermöglicht eine Verflüssigung des gasförmigen Kohlendioxids. Die Entgasungsanlage, bevorzugt ein Flash-Drum oder Gas-Flüssig Separator ermöglicht die Entfernung/Reduzierung von inerten Gasen, beispielsweise Argon und Stickstoff. Eine Pumpe ist mit der Entgasungsanlage verbunden und ermöglicht eine Druckerhöhung des flüssigen Kohlendioxids auf bevorzugt 100 bar bis 250 bar, besonders bevorzugt 150 bar bis 200 bar. Der Ausdruck "Pumpe", beispielsweise eine Plunger Pumpe, umfasst im Sinne der Erfindung dem Fachmann bekannte Pumpen (und Kompressoren) zur Druckerhöhung von Flüssigkeiten (und/oder Gasen) und/oder überkritischen Fluiden und/oder Gemischen davon. Eine dritte Verbindungsleitung ist mit der Pumpe und der Zuleitung (der Anlagenkomplex zur Beschichtung von harnstoffhaltigen Granulat) für überkritisches Kohlendioxid verbunden. Bevorzugt durch Druck- und/oder Temperaturerhöhung, erfolgt in der dritten Verbindungsleitung und in den angeschlossenen Bereichen eine Umwandlung des flüssigen Kohlendioxids in überkritisches Kohlendioxid. Besonders bevorzugt wird nur die Temperatur erhöht. Hierzu können bevorzugt in der dritten Verbindungsleitung beispielsweise Wärmetauscher und/oder Heizungen vorgesehen sein. Bevorzugt wird die Kälte des flüssigen CO₂ nach der Abtrennung von Inerten in der Entgasungsanlage und nach der Verdichtung mit der Pumpe für die weitere Abkühlung des Gases (CO₂) vor der Ansaugstelle der zweiten Kompressionsstufe verwendet (nach dem prozesseigenem Kühler, z.B. Interstage Cooler). Bevorzugt wird das vorgewärmte verflüssigte CO₂ für die weitere Abkühlung des Gases vor der Ansaugstelle der ersten Kompressionsstufe verwendet (nach dem prozesseigenem Kühler) und wird anschließend unter Verwendung der Wärme des Stoffstroms nach der zweiten Kompressionsstufe auf die Zieltemperatur gebracht. Die Reihenfolge der Kälteintegration (beispielsweise durch Kühler, Wärmetauscher, Heizungen) kann nach Bedarf auch verändert werden, d.h. zuerst vor den ersten Kompressionsstufen und danach vor der zweiten Kompressionsstufe.

In einer bevorzugten Ausführungsform sind Elemente zur Kälte-/Wärmerückgewinnung und/oder Kühler innerhalb der Ammoniaksynthese-Anlage und Harnstoffsynthese-Anlage)(Ammoniak-Harnstoff-Komplex) bei einer oder mehrerer der folgenden Elemente umfasst:
- der Kohlendioxid CO₂-Zuleitung;
- zwischen der ersten Kompressionsstufe und der zweiten Kompressionsstufe;
- zwischen der zweiten Kompressionsstufe und der dritten Kompressionsstufe;
- zwischen der dritten Kompressionsstufe und der vierten Kompressionsstufe; und/oder
- der ersten Verbindungsleitung.

Der Ausdruck "Kälte-ZWärmerückgewinnung" umfasst im Sinne der Erfindung dem Fachmann gebräuchliche Wärmetauscher, Kühler, Heizungen und ähnliche Vorrichtungen. In Abhängigkeit von der genauen Auslegung des Verfahrens sind eine gezielte Temperaturkontrolle und die Rückgewinnung von überschüssigen Wärmemengen möglich. Diese Rückgewinnung senkt die Prozesskosten und benötigten Prozessmittel.

Bevorzugt ist zwischen der zweiten Kompressionsstufe und der dritten Kompressionsstufe eine Passivierungsluftzuleitung angeordnet, welche beispielsweise in einem nachgeschaltetem Wasserstoff H₂-Removal-Reaktor und in der Harnstoff-Synthese benötigt wird. Beispielsweise stellt die Passivierungsluft Sauerstoff für die katalytische Reaktion (7) 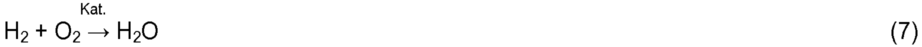 und für die Bildung der Oxidschicht im Hochdruck- und Hochtemperaturbereich der Harnstoff-Synthese bereit. Die Passivierungsluftzuleitung ist in Prozessrichtung hinter der Abzweigung zur Entnahme des gasförmigen CO₂ für die anschließende Umwandlung in überkritisches CO₂ zwischen der zweiten Kompressionsstufe und der dritten Kompressionsstufe angeordnet. Dies vermeidet eine Kontaminierung des für die Bildung des überkritischen CO₂ abgezweigten Gasstroms.

Die Erfindung umfasst des Weiteren die Verwendung des erfindungsgemäßen Anlagenkomplexes zur Beschichtung von harnstoffhaltigen Partikeln mit bioabbaubaren Polymeren.

Des Weiteren wird die Erfindung anhand der folgenden Figuren näher erläutert. Die Figuren beschränken dabei nicht den Schutzumfang der Erfindung, sondern dienen nur der beispielhaften Erläuterung. Die Figuren sind nicht maßstabsgetreu.

Es zeigen:
Figur 1 einen schematischen Querschnitt des erfindungsgemäßen Anlagenkomplexes zur Beschichtung von harnstoffhaltigen Granulat und
Figur 2 einen weiteren schematischen Querschnitt des erfindungsgemäßen Anlagenkomplexes zur Beschichtung von harnstoffhaltigen Granulat und einen Ausschnitt aus der Ammoniak-Harnstoff-Komplex.

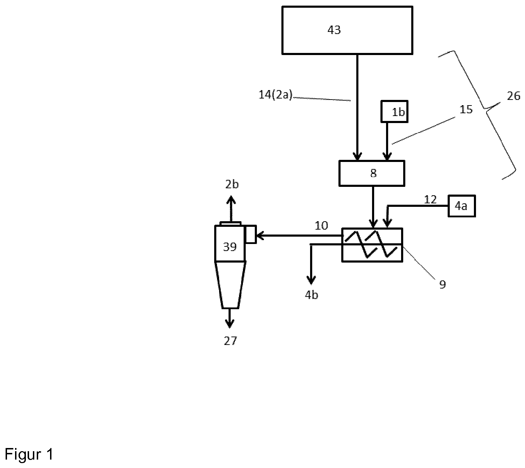

Figur 1 zeigt einen schematischen Querschnitt des erfindungsgemäßen Anlagenkomplexes zur Beschichtung von harnstoffhaltigen Granulat mindestens umfassend ein Ammoniak-Harnstoff-Komplex (43) und eine Anlage zur Beschichtung von harnstoffhaltigen Granulat (26). Das in dem Ammoniak-Harnstoff-Komplex (43) erhaltene überkritische Kohlendioxid (2a) gelangt über die Zuleitung für überkritisches Kohlendioxid (14) aus dem Ammoniak-Harnstoff-Komplex (43) zusammen mit einer Zuleitung (15) für ein bioabbaubares Polymer (1b) in eine Mischvorrichtung (8). Eine Zuleitung für ein harnstoffhaltiges Granulat (13) und eine Zuleitung aus der Mischvorrichtung (8) mit dem im überkritischen Kohlendioxid gelösten bioabbaubaren Polymer (1b) sind mit einem Beschichter (9), beispielsweise Trommelcoater, Wirbelschichtcoater, und/oder Wirbelschichtcoater mit tangentialem Sprühen, verbunden. Der Beschichter umfasst einen ersten Ausgang (10) für gasförmiges Kohlendioxid (2b) und einen zweiten Ausgang für das beschichtete Produkt (4b). Der erste Ausgang (10) für gasförmiges Kohlendioxid (2b) ist mit einem Staubwäscher, Staubabscheider und/oder Zyklon (39) verbunden. Die aufgefangenen Staubpartikel (27) können alternativ entsorgt oder in den Prozess zurückgeführt werden. Der Staub besteht zum (voraussichtlich) größten Teil aus Polymerpartikeln. Diese werden bevorzugt (nicht gezeigt) mit Wasser gewaschen, anschließend eingedampft und in die Mischvorrichtung (8) zurückgeführt. Diese Eindampfung kann in der Harnstoffsynthese-Anlage erfolgen. Dieser Strom ist tendenziell eher klein und kann der Haupteindampfung einfach beigemischt werden. Das Polymer wird sich eher nicht lösen, so dass keine Eindampfung, sondern evtl. eine Trocknung erforderlich wird.

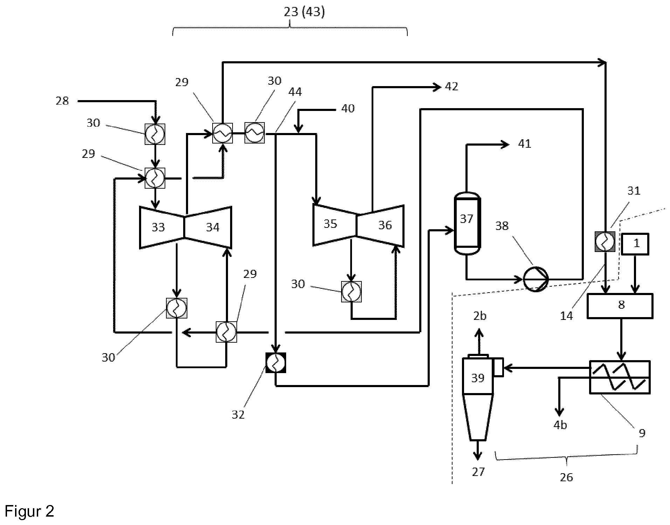

Figur 2 zeigt einen weiteren schematischen Querschnitt des erfindungsgemäßen Anlagenkomplexes zur Beschichtung von harnstoffhaltigen Granulat (26) und einen Ausschnitt aus der Ammoniak-Harnstoff-Komplex (43). Kohlendioxid, bevorzugt Kohlendioxid aus der Kohlendioxidwäsche des Steamreforming-Prozesses, wird in die gezeigte Kohlendioxid-Komprimierungsstufe innerhalb der Ammoniak-Harnstoff-Komplex (43) über mindestens eine Kohlendioxid CO₂-Zuleitung (28) in eine (mit von der ersten zur vierten Stufe ansteigenden Druck) erste Kompressionsstufe (33) (bevorzugte Druckbereiche von 5 bar bis 10 bar), einer zweiten Kompressionsstufe (34) (bevorzugte Druckbereiche von 20 bar bis 30 bar), einer dritten Kompressionsstufe (35) (bevorzugte Druckbereiche von 70 bar bis 90 bar) und eine vierte Kompressionsstufe (36) (bevorzugte Druckbereiche von 140 bar bis 180 bar) eingeleitet. Eine erste Verbindungsleitung (42) ist aus der vierten Kompressionsstufe (36) mit einer Harnstoffsynthese-Einheit verbunden. Der Ausdruck "Harnstoffsynthese-Einheit" umfasst im Sinne der Erfindung die innerhalb der Harnstoffsynthese-Anlage vorgesehen Reaktoren und Zuleitungen zur eigentlichen Harnstoffsynthese aus CO₂ und NH₃. Diese Verbindung ermöglicht eine Verwendung des komprimierten Kohlendioxids in der Synthese von Harnstoff. Der erfindungsgemäße Anlagenkomplex umfasst eine Abzweigung (44) zwischen der zweiten Kompressionsstufe (34) und der dritten Kompressionsstufe (35). Ausgehend von der Abzweigung (44) führt eine zweite Verbindungsleitung zu einer Entgasungsanlage (37), wobei die zweite Verbindungsleitung vor der Entgasungsanlage (37) eine Kälteanlage (32) durchläuft (durchgehend verbunden ist). Die Kälteanlage (32) ermöglicht eine Verflüssigung des gasförmigen Kohlendioxids. Die Entgasungsanlage (37) ermöglicht die Entfernung/Reduzierung von inerten Gasen, beispielsweise Argon, nicht kondensierbare Anteile von CO₂ und Stickstoff über die Abluftleitung (41). Eine Pumpe (38) ist mit der Entgasungsanlage verbunden und ermöglicht eine Druckerhöhung des flüssigen Kohlendioxids auf 150 bar bis 200 bar. Eine dritte Verbindungsleitung ist mit der Pumpe (38) und der Zuleitung (14) für überkritisches Kohlendioxid verbunden. Bevorzugt durch Temperaturerhöhung, erfolgt in der dritten Verbindungsleitung und in den angeschlossenen Bereichen eine Umwandlung des flüssigen Kohlendioxids (2c) in überkritisches Kohlendioxid (2a). Elemente zur Kälte-/Wärmerückgewinnung (29) und/oder Kühler (30) und/oder Heizungen (31) sind bei den folgenden Elementen umfasst: der Kohlendioxid CO₂-Zuleitung (28), zwischen der ersten Kompressionsstufe (33) und der zweiten Kompressionsstufe (34), zwischen der zweiten Kompressionsstufe (34) und der dritten Kompressionsstufe (35), zwischen der dritten Kompressionsstufe (35) und der vierten Kompressionsstufe (36); und/oder der ersten Verbindungsleitung (42) zur Harnstoffsyntheseanlage. Bevorzugt gehört der in Flussrichtung der CO₂- Zuleitung (28) erste Kühler (30) zur Ammoniaksynthese-Anlage, die restlichen Elemente zur Erzeugung des überkritischen Kohlendioxids zur Harnstoffsynthese-Anlage. Zwischen der zweiten Kompressionsstufe (34) und der dritten Kompressionsstufe (35) ist eine Passivierungsluftzuleitung (40) angeordnet, welche beispielsweise in einem (nicht gezeigten) nachgeschaltetem Wasserstoff H₂-Removal-Reaktor und in der Harnstoff-Synthese benötigt wird. Über die Zuleitung für überkritisches Kohlendioxid (14) schließt sich der in Figur 1 beschriebene Aufbau an.

### Bezugszeichenliste

- (1): organisches Polymer
- (1b): biologisch abbaubares Polymer
- (2a): überkritisches Kohlendioxid
- (2b): gasförmiges Kohlendioxid
- (3): polymerhaltige Lösung
- (4a): harnstoffhaltiges Granulat
- (4b): beschichtetes harnstoffhaltiges Granulat
- (8): Mischvorrichtung
- (9): Beschichter
- (10): Ausgang für gasförmiges Kohlendioxid
- (11): Ausgang für beschichtetes Produkt
- (12): Zuleitung für harnstoffhaltiges Granulat
- (14): Zuleitung für überkritisches Kohlendioxid
- (15): Zuleitung (15) für das bioabbaubaren Polymer
- (23): Kohlendioxid-Komprimierungsstufe
- (26): Anlage zur Beschichtung von harnstoffhaltigen Partikeln/Granulat
- (27): Staubpartikel
- (28): CO₂-Zuleitung (28) verbunden mit einer ersten Kompressionsstufe
- (29): Elemente zur Kälte-/Wärmerückgewinnung (29)
- (30): Kühler
- (31): Heizung/Heizelement
- (32): Kälteanlage/Kühler
- (33): ersten Kompressionsstufe
- (34): zweite Kompressionsstufe
- (35): dritte Kompressionsstufe
- (36): vierte Kompressionsstufe
- (37): Entgasungsanlage
- (38): Pumpe
- (39): Staubwäscher, Staubabscheider, sauren Wäscher und/oder Zyklon
- (40): Passivierungsluftzuleitung
- (41): Abluftleitung
- (42): erste Verbindungsleitung zu einer Harnstoffsynthese-Einheit
- (43): Ammoniak-Harnstoff-Komplex

## Patentansprüche

1. Verfahren zur Beschichtung von harnstoffhaltigen Granulat mit organischen Polymeren mindestens umfassend:
a.) komprimieren und kondensieren von gasförmigen Kohlendioxid (2b) und erhalten von flüssigen Kohlendioxid;
b.) Erhöhung des Drucks und/oder der Temperatur über den kritischen Punkt von Kohlendioxid und erhalten von überkritischen Kohlendioxid (2a);
c.) lösen eines organischen Polymers (1) in dem überkritischen Kohlendioxid (2a) und erhalten einer polymerhaltigen Lösung (3),
d.) Vermischen, Aufsprühen und/oder in Kontaktbringen der polymerhaltigen Lösung (3) mit einem harnstoffhaltigen Granulat (4a); wobei
- eine gleichzeitige oder anschließende Erniedrigung der Temperatur und/oder des Drucks unterhalb des kritischen Punktes von Kohlendioxid erfolgt; und
- ein beschichtetes harnstoffhaltiges Granulat (4b) und gasförmigen Kohlendioxid (2b) erhalten werden,
wobei das harnstoffhaltige Granulat (4a) eine mittlere Partikelgröße von 0,5 mm bis 8 mm, bevorzugt 1 mm bis 6 mm, besonders bevorzugt 2 mm bis 4 mm aufweist.

2. Verfahren nach Anspruch 1, **dadurch gekennzeichnet, dass** das organische Polymer (1) biologisch abbaubare Polymere (1b) umfasst.

3. Verfahren nach Anspruch 2, **dadurch gekennzeichnet, dass** das biologisch abbaubare Polymer (1b) Polylactide (PLA), Polyglykole, Polycaprolactone, Poly(hydroxybuttersäure), Poly(hydroxyvalerinsäure), Polyalkylterephthalate, Polyanhydride, Poly(1,4-dioxan-2,5-dion), Polyaminosäuren, Polysaccharide, Cellulosester, Cellulosehydrat, Celluloseacetat, Carboxymethylcellulose, Lignin, Polyhydroxyfettsäuren, Stärke, biologisch abbaubare Polyester, biologisch abbaubare Polyamide, biologisch abbaubare Polyimide, Polyhydroxyalkanoate und Polybuthylensuccinate (PBS), Amylose, Amylopektin und/oder Gemische, Oligomere, Derivate und Copolymere davon umfasst.

4. Verfahren nach einem der Ansprüche 1 bis 3, **dadurch gekennzeichnet, dass** die polymerhaltige Lösung größer 20 Gew. % bis 70 Gew. % biologisch abbaubare Polymere (1b) enthält, bevorzugt 40 Gew. % bis 60 Gew. % biologisch abbaubare Polymere (1b).

5. Verfahren nach einem der Ansprüche 1 bis 4, **dadurch gekennzeichnet, dass** das gasförmige Kohlendioxid (2b) ins Verfahren zurückgeführt und in überkritisches Kohlendioxid (2a) umgewandelt wird.

6. Verfahren nach einem der Ansprüche 1 bis 5, **dadurch gekennzeichnet, dass** das gasförmige Kohlendioxid und das überkritische Kohlendioxid (2a) in einem verbundenen industrielen Anlagenkomplex (26a) bereitgestellt werden, bevorzugt in einer Harnstoffsynthese-Anlage, Kokereianlage, Raffinerieanlagenund/oder in einem Ammoniak-Harnstoff-Komplex, bevorzugt in einem Ammoniak-Harnstoff-Komplex.

7. Verfahren nach einem der Ansprüche 1 bis 6, **dadurch gekennzeichnet, dass** das Kondensieren in Schritt a.) mithilfe eines Kühlmittels, insbesondere Ammoniak, aus einer angeschlossenen Ammoniak-Kälteanlage erfolgt.

8. Verfahren nach einem der Ansprüche 1 bis 7, **dadurch gekennzeichnet, dass** das flüssige Kohlendioxid nach Schritt a.) einen Flash Verfahren für die Entfernung und/oder Ausgasen Inerter und nicht kondensierbarer Gase unterzogen wird.

9. Verfahren nach einem der Ansprüche 1 bis 7, **dadurch gekennzeichnet, dass** das Komprimieren des gasförmigen Kohlendioxids (2b) in einer ersten Stufe A in einer LP(Niederdruck)-Kompressionsstufe (33) und in einer zweiten Stufe B in einer HP(Hochdruck)-Kompressionsstufe (34) erfolgt.

10. Verfahren nach Anspruch 9, **dadurch gekennzeichnet, dass** eine Kälte-/Wärmerückgewinnung (29) und/oder Kühler (30) vorgesehen sind:
- vor der ersten Stufe A;
- zwischen der ersten Stufe A und der zweiten Stufe B; und/oder
- nach der zweiten Stufe B.

11. Verfahren nach einem der Ansprüche 1 bis 10, **dadurch gekennzeichnet, dass** das harnstoffhaltige Granulat (4a) und/oder das beschichtete harnstoffhaltige Granulat (4b) Ammoniumsalze, Nitrate, Phosphate, Schwefel, Kalium, Kalzium, bevorzugt Harnstoff, Ammoniumsulfat, Ammoniumnitrat, Phosphate, Schwefel und/oder Gemische davon enthält.

12. Verfahren nach einem der Ansprüche 1 bis 11, **dadurch gekennzeichnet, dass** das Vermischen der polymerhaltigen Lösung (3) mit dem harnstoffhaltigen Granulat (4a) in einem oder mehreren Beschichtern (9), bevorzugt Trommelcoater, Wirbelschichtcoater, und/oder Wirbelschichtcoater mit tangentialem Sprühen erfolgt.

13. Anlagenkomplex zur Beschichtung von harnstoffhaltigen Granulat mit biologisch abbaubaren Polymeren mindestens umfassend eine Harnstoffsynthese-Anlage und eine Anlage (26) zur Beschichtung von harnstoffhaltigen Granulat, wobei die Anlage (26) zur Beschichtung von harnstoffhaltigen Granulat mindestens umfasst:
a.) Eine Zuleitung (14) für überkritisches Kohlendioxid (2a) aus der Harnstoffsynthese-Anlage und eine Zuleitung (15) für ein biologisch abbaubares Polymer (1b)
b.) eine Mischvorrichtung (8) für überkritisches Kohlendioxid (2a) und das biologisch abbaubare Polymers (1b), welche mit der Zuleitung (14) für das überkritische Kohlendioxid (2a) und der Zuleitung (15) für das biologisch abbaubare Polymer (1b) verbunden ist;
c.) einen Beschichter (9) verbunden mit der Mischvorrichtung (8) und einer Zuleitung (12) für ein Granulat (13), wobei der Beschichter (9) einen ersten Ausgang (10) für gasförmiges Kohlendioxid (2b) und einen zweiten Ausgang (11) für ein beschichtetes Produkt (4b) aufweist,
wobei der erste Ausgang (10) für gasförmiges Kohlendioxid (2b) mit einem Staubwäscher, Staubabscheider und/oder Zyklon (39) verbunden ist.

14. Anlagenkomplex nach Anspruch 13, **dadurch gekennzeichnet, dass** die Harnstoffsynthese-Anlage mit einer Ammoniak-Syntheseeinheit als Ammoniak-Harnstoff-Komplex und/oder einer Harnstoffgranulationsanlage verbunden ist.

15. Anlagenkomplex nach einem der Ansprüche 13 oder 14, **dadurch gekennzeichnet, dass** die Kohlendioxid-Komprimierungsstufe (23) mindestens umfasst:
- eine CO₂-Zuleitung (28) verbunden mit einer ersten Kompressionsstufe (33);
- eine zweite Kompressionsstufe (34) verbunden mit der ersten Kompressionsstufe (33);
- eine dritte Kompressionsstufe (35) verbunden mit der zweiten Kompressionsstufe (34);
- eine vierte Kompressionsstufe (36) verbunden mit der dritten Kompressionsstufe (35);
- eine erste Verbindungsleitung zu einer Harnstoffsynthese-Einheit (42) verbunden mit der vierten Kompressionsstufe (36);
- eine Abzweigung (44) zwischen der zweiten Kompressionsstufe (34) und der dritten Kompressionsstufe (35) und eine zweite Verbindungsleitung von der Abzweigung (44) zu einer Entgasungsanlage (37), wobei die zweite Verbindungsleitung eine Kälteanlage (32) durchläuft;
- eine Pumpe (38) verbunden mit der Entgasungsanlage (37);
- eine dritte Verbindungsleitung verbunden mit der Pumpe (38) und der Zuleitung (14) für überkritisches Kohlendioxid (2a).

16. Anlagenkomplex nach Anspruch 15, **dadurch gekennzeichnet, dass** Elemente zur Kälte-/Wärmerückgewinnung (29) und/oder Kühler (30) innerhalb der Harnstoffsynthese-Anlage oder des Ammoniak-Harnstoff-Komplexes bei einer oder mehrerer der folgende Elemente umfasst sind:
- der CO₂-Zuleitung (28);
- zwischen der ersten Kompressionsstufe (33) und der zweiten Kompressionsstufe (34);
- zwischen der zweiten Kompressionsstufe (34) und der dritten Kompressionsstufe (35);
- zwischen der dritten Kompressionsstufe (35) und der vierten Kompressionsstufe (36); und/oder
- die erste Verbindungsleitung.

17. Anlagenkomplex nach einem der Ansprüche 15 oder 16, **dadurch gekennzeichnet, dass** zwischen der zweiten Kompressionsstufe (34) und der dritten Kompressionsstufe (35) eine Passivierungsluftzuleitung (40) angeordnet ist.

18. Verwendung des Anlagenkomplexes nach einem der Ansprüche 13 bis 17 zur Beschichtung von harnstoffhaltigen Partikeln mit bioabbaubaren Polymeren.

## Claims

1. A process for the coating of urea-containing granules with organic polymers at least including:
a.) compressing and condensing gaseous carbon dioxide (2b) and obtaining liquid carbon dioxide;
b.) increasing the pressure and/or the temperature above the critical point of carbon dioxide and obtaining supercritical carbon dioxide (2a);
c.) dissolving an organic polymer (1) in the supercritical carbon dioxide (2a) and obtaining a polymer-containing solution (3),
d.) mixing the polymer-containing solution (3) with, spraying it onto and/or bringing it into contact with urea-containing granules (4a); in which
- a simultaneous or subsequent lowering of the temperature and/or of the pressure below the critical point of carbon dioxide takes place; and
- coated urea-containing granules (4b) and gaseous carbon dioxide (2b) are obtained,
wherein the urea-containing granules (4a) exhibit a mean particle size of 0.5 mm to 8 mm, preferably 1 mm to 6 mm, particularly preferably 2 mm to 4 mm.

2. The process as claimed in claim 1, **characterized in that** the organic polymer (1) includes biodegradable polymers (1b).

3. The process as claimed in claim 2, **characterized in that** the biodegradable polymer (1b) includes polylactides (PLA), polyglycols, polycaprolactones, poly(hydroxybutyric acid), poly(hydroxyvaleric acid), polyalkyl terephthalates, polyanhydrides, poly(1,4-dioxane-2,5-dione), polyamino acids, polysaccharides, cellulose esters, cellulose hydrate, cellulose acetate, carboxymethyl cellulose, lignin, polyhydroxy fatty acids, starch, biodegradable polyesters, biodegradable polyamides, biodegradable polyimides, polyhydroxyalkanoates and polybutylene succinates (PBS), amylose, amylopectin and/or mixtures, oligomers, derivatives and copolymers thereof.

4. The process as claimed in one of claims 1 to 3, **characterized in that** the polymer-containing solution contains greater 20 wt. % to 70 wt. % biodegradable polymers (1b), preferably 40 wt. % to 60 wt. % biodegradable polymers (1b).

5. The process as claimed in one of claims 1 to 4, **characterized in that** the gaseous carbon dioxide (2b) is returned to the process and converted into supercritical carbon dioxide (2a).

6. The process as claimed in one of claims 1 to 5, **characterized in that** the gaseous carbon dioxide and the supercritical carbon dioxide (2a) are provided in a connected industrial plant complex (26a), preferably in a urea synthesis plant, coking plant, refinery plant and/or in an ammonia-urea complex, preferably in an ammonia-urea complex.

7. The process as claimed in one of claims 1 to 6, **characterized in that** the condensation in stage a.) takes place with the aid of a coolant, in particular ammonia, from a connected ammonia refrigerating system.

8. The process as claimed in one of claims 1 to 7, **characterized in that** the liquid carbon dioxide is subjected, after step a.), to a flash process for the removal and/or outgassing of inert and noncondensable gases.

9. The process as claimed in one of claims 1 to 7, **characterized in that** the compression of the gaseous carbon dioxide (2b) takes place in a first step A in a LP (low-pressure) compression step (33) and in a second step B in an HP (high-pressure) compression step (34).

10. The process as claimed in claim 9, **characterized in that** a cold/heat recovery (29) and/or cooler (30) are provided:
- before the first step A;
- between the first step A and the second step B; and/or
- after the second step B.

11. The process as claimed in one of claims 1 to 10, **characterized in that** the urea-containing granules (4a) and/or the coated urea-containing granules (4b) contain ammonium salts, nitrates, phosphates, sulfur, potassium, calcium, preferably urea, ammonium sulfate, ammonium nitrate, phosphates, sulfur and/or mixtures thereof.

12. The process as claimed in one of claims 1 to 11, **characterized in that** the polymer-containing solution (3) is mixed with the urea-containing granules (4a) in one or more coaters (9), preferably drum coaters, fluidized bed coaters, and/or fluidized bed coaters with tangential spraying.

13. A plant complex for the coating of urea-containing granules with biodegradable polymers at least including a urea synthesis plant and a plant (26) for the coating of urea-containing granules, the plant (26) for the coating of urea-containing granules including at least:
a.) a feed line (14) for supercritical carbon dioxide (2a) from the urea synthesis plant and a feed line (15) for a biodegradable polymer (1b);
b.) a mixing device (8) for supercritical carbon dioxide (2a) and the biodegradable polymer (1b), which is connected to the feed line (14) for the supercritical carbon dioxide (2a) and to the feed line (15) for the biodegradable polymer (1b);
c.) a coater (9) connected to the mixing device (8) and a feed line (12) for granules (13), the coater (9) exhibiting a first outlet (10) for gaseous carbon dioxide (2b) and a second outlet (11) for a coated product (4b),
wherein the first outlet (10) for gaseous carbon dioxide (2b) is connected to a dust scrubber, dust separator and/or cyclone (39).

14. The plant complex as claimed in claim 13, **characterized in that** the urea synthesis plant is connected to an ammonia synthesis unit as ammonia-urea complex and/or to a urea granulation plant.

15. The plant complex as claimed in one of claims 13 or 14, **characterized in that** the carbon dioxide compression step (23) includes at least:
- a CO₂ feed line (28) connected to a first compression step (33);
- a second compression step (34) connected to the first compression step (33);
- a third compression step (35) connected to the second compression step (34);
- a fourth compression step (36) connected to the third compression step (35);
- a first connecting line to a urea synthesis unit (42) connected to the fourth compression step (36);
- a branch (44) between the second compression step (34) and the third compression step (35) and a second connecting line from the branch (44) to a degassing system (37), the second connecting line running through a refrigerating system (32);
- a pump (38) connected to the degassing system (37);
- a third connecting line connected to the pump (38) and to the feed line (14) for supercritical carbon dioxide (2a).

16. The plant complex as claimed in claim 15, **characterized in that** elements for cold/heat recovery (29) and/or coolers (30) are included within the urea synthesis plant or the ammonia-urea complex in one or more of the following elements:
- the CO₂ feed line (28);
- between the first compression step (33) and the second compression step (34);
- between the second compression step (34) and the third compression step (35);
- between the third compression step (35) and the fourth compression step (36); and/or
- the first connecting line.

17. The plant complex as claimed in either of claims 15 and 16, **characterized in that** a passivation air supply line (40) is arranged between the second compression step (34) and the third compression step (35).

18. The use of the plant complex as claimed in one of claims 13 to 17 for the coating of urea-containing particles with biodegradable polymers.

## Revendications

1. Procédé d'enrobage de granulés contenant de l'urée avec des polymères organiques comprenant au moins :
a.) comprimer et condenser le dioxyde de carbone gazeux (2b) et obtenir du dioxyde de carbone liquide ;
b.) en augmentant la pression et/ou la température au-dessus du point critique du dioxyde de carbone et en obtenant du dioxyde de carbone supercritique (2a) ;
c.) dissolution d'un polymère organique (1) dans le dioxyde de carbone supercritique (2a) et obtention d'une solution contenant le polymère (3),
d.) mélanger la solution contenant des polymères (3) avec des granulés contenant de l'urée (4a), la pulvériser sur ces granulés et/ou la mettre en contact avec eux ; dans ce cas
- un abaissement simultané ou ultérieur de la température et/ou de la pression au-dessous du point critique du dioxyde de carbone a lieu ; et
- on obtient des granulés enrobés d'urée (4b) et du dioxyde de carbone gazeux (2b),
dans lequel les granulés contenant de l'urée (4a) présentent une taille moyenne de 0,5 mm à 8 mm, de préférence de 1 mm à 6 mm, de préférence encore de 2 mm à 4 mm.

2. Procédé selon la revendication 1, **caractérisé par le fait que** le polymère organique (1) comprend des polymères biodégradables (1b).

3. Procédé selon la revendication 2, **caractérisé par le fait que** le polymère biodégradable (1b) comprend les polylactides (PLA), les polyglycols, les polycaprolactones, le poly(acide hydroxybutyrique), le poly(acide hydroxyvalérique), les polyalkyl téréphtalates, les polyanhydrides, le poly(1,4-dioxane-2,5-dione), les polyaminoacides, les polysaccharides, les esters de cellulose, l'hydrate de cellulose, l'acétate de cellulose, la carboxyméthylcellulose, la lignine, les acides gras polyhydroxylés, l'amidon, les polyesters biodégradables, les polyamides biodégradables, les polyimides biodégradables, les polyhydroxyalcanoates et les succinates de polybutylène (PBS), l'amylose, l'amylopectine et/ou les mélanges, oligomères, dérivés et copolymères de ces derniers.

4. Procédé selon l'une des revendications 1 à 3, **caractérisé par le fait que** la solution contenant le polymère contient plus de 20 % en poids à 70 % en poids de polymères biodégradables (1b), de préférence 40 % en poids à 60 % en poids de polymères biodégradables (1b).

5. Procédé selon l'une des revendications 1 à 4, **caractérisé par le fait que** le dioxyde de carbone gazeux (2b) est renvoyé dans le procédé et converti en dioxyde de carbone supercritique (2a).

6. Procédé selon l'une des revendications 1 à 5, **caractérisé par le fait que** le dioxyde de carbone gazeux et le dioxyde de carbone supercritique (2a) sont fournis dans un complexe industriel connecté (26a), de préférence dans une usine de synthèse d'urée, une cokerie, une raffinerie et/ou dans un complexe ammoniac-urée, de préférence dans un complexe ammoniac-urée.

7. Procédé selon l'une des revendications 1 à 6, **caractérisé par le fait que** la condensation à l'étape a.) a lieu à l'aide d'un réfrigérant, en particulier de l'ammoniac, provenant d'un système de réfrigération à l'ammoniac raccordé.

8. Procédé selon l'une des revendications 1 à 7, **caractérisé par le fait que** le dioxyde de carbone liquide est soumis, après l'étape a.), à un procédé de flash pour l'élimination et/ou le dégazage des gaz inertes et incondensables.

9. Procédé selon l'une des revendications 1 à 7, **caractérisé par le fait que** la compression du dioxyde de carbone gazeux (2b) a lieu dans une première étape A dans une étape de compression LP (basse pression) (33) et dans une deuxième étape B dans une étape de compression HP (haute pression) (34).

10. Procédé selon la revendication 9, **caractérisé par la** présence d'un récupérateur de froid/chaleur (29) et/ou d'un refroidisseur (30) :
- avant la première étape A ;
- entre la première étape A et la deuxième étape B ; et/ou
- après la deuxième étape B.

11. Procédé selon l'une des revendications 1 à 10, **caractérisé par le fait que** les granulés contenant de l'urée (4a) et/ou les granulés enrobés contenant de l'urée (4b) contiennent des sels d'ammonium, des nitrates, des phosphates, du soufre, du potassium, du calcium, de préférence de l'urée, du sulfate d'ammonium, du nitrate d'ammonium, des phosphates, du soufre et/ou des mélanges de ceux-ci.

12. Procédé selon l'une des revendications 1 à 11, **caractérisé par le fait que** la solution contenant du polymère (3) est mélangée aux granulés contenant de l'urée (4a) dans un ou plusieurs enrobeurs (9), de préférence des enrobeurs à tambour, des enrobeurs à lit fluidisé, et/ou des enrobeurs à lit fluidisé avec pulvérisation tangentielle.

13. Complexe d'installations pour l'enrobage de granulés contenant de l'urée avec des polymères biodégradables, comprenant au moins une installation de synthèse d'urée et une installation (26) pour l'enrobage de granulés contenant de l'urée, l'installation (26) pour l'enrobage de granulés contenant de l'urée comprenant au moins :
a.) une conduite d'alimentation (14) pour le dioxyde de carbone supercritique (2a) provenant de l'usine de synthèse d'urée et une conduite d'alimentation (15) pour un polymère biodégradable (1b) ;
b.) un dispositif de mélange (8) pour le dioxyde de carbone supercritique (2a) et le polymère biodégradable (1b), qui est connecté à la conduite d'alimentation (14) pour le dioxyde de carbone supercritique (2a) et à la conduite d'alimentation (15) pour le polymère biodégradable (1b) ;
c.) un dispositif d'enrobage (9) connecté au dispositif de mélange (8) et à une ligne d'alimentation (12) pour les granulés (13), le dispositif d'enrobage (9) présentant une première sortie (10) pour le dioxyde de carbone gazeux (2b) et une seconde sortie (11) pour un produit enrobé (4b),
dans lequel la première sortie (10) pour le dioxyde de carbone gazeux (2b) est reliée à un épurateur de poussières, un séparateur de poussières et/ou un cyclone (39).

14. Complexe d'installations selon la revendication 13, **caractérisé par le fait que** l'usine de synthèse d'urée est reliée à une unité de synthèse d'ammoniac en tant que complexe ammoniac-urée et/ou à une usine de granulation d'urée.

15. Complexe d'installations selon l'une des revendications 13 ou 14, **caractérisé par le fait que** l'étape de compression du dioxyde de carbone (23) comprend au moins:
- une ligne d'alimentation en CO₂ (28) reliée à une première étape de compression (33) ;
- une deuxième étape de compression (34) reliée à la première étape de compression (33) ;
- une troisième étape de compression (35) reliée à la deuxième étape de compression (34) ;
- une quatrième étape de compression (36) reliée à la troisième étape de compression (35) ;
- une première ligne de raccordement à une unité de synthèse d'urée (42) reliée à la quatrième étape de compression (36) ;
- une dérivation (44) entre la deuxième étape de compression (34) et la troisième étape de compression (35) et une deuxième conduite de raccordement de la dérivation (44) à un système de dégazage (37), la deuxième conduite de raccordement traversant un système de réfrigération (32) ;
- une pompe (38) reliée au système de dégazage (37) ;
- une troisième conduite de raccordement reliée à la pompe (38) et à la conduite d'alimentation (14) en dioxyde de carbone supercritique (2a).

16. Complexe d'installations selon la revendication 15, **caractérisé par le fait que des** éléments pour la récupération du froid/de la chaleur (29) et/ou des refroidisseurs (30) sont inclus dans l'usine de synthèse d'urée ou le complexe ammoniac-urée dans un ou plusieurs des éléments suivants :
- la ligne d'alimentation CO₂ (28) ;
- entre la première étape de compression (33) et la deuxième étape de compression (34) ;
- entre la deuxième étape de compression (34) et la troisième étape de compression (35) ;
- entre la troisième étape de compression (35) et la quatrième étape de compression (36) ; et/ou
- la première ligne de connexion.

17. Complexe d'installations selon l'une des revendications 15 et 16, **caractérisé par le fait qu'**une conduite d'alimentation en air de passivation (40) est disposée entre la deuxième étape de compression (34) et la troisième étape de compression (35).

18. Utilisation du complexe d'installations selon l'une des revendications 13 à 17 pour l'enrobage de particules contenant de l'urée avec des polymères biodégradables.
